# EUROPEAN PATENT SPECIFICATION

(11) **EP 4 543 516 B1**
(45) Date of publication and mention of the grant of the patent: **29.04.2026**
(21) Application number: 23751343.7
(22) Date of filing: 20.06.2023
(51) Int. Cl.: A61M 16/00

(54) **APPARATUS FOR ADMINISTERING GAS TO PATIENTS**
VORRICHTUNG ZUR VERABREICHUNG VON GAS AN PATIENTEN
APPAREIL D'ADMINISTRATION DE GAZ À DES PATIENTS

(30) Priority: 21.06.2022 GB 202209084
(43) Date of publication of application: 30.04.2025
(73) Proprietor: Elegant Design and Solutions Ltd, Uckfield East Sussex TN22 3RX (GB)
(72) Inventor: PENNINGTON-RIDGE, Edward John, Uckfield East Sussex TN22 3RX (GB)
(74) Representative: K2 IP Limited
(86) International application number: PCT/GB2023/051609
(87) International publication number: WO 2023/247944

(56) References cited:
- WO-A1-2016/085807
- WO-A1-2017/006186

## Description

### Field of the Invention

This invention relates to an apparatus for administering gas to patients, particularly those in need of pain relief. It has been developed primarily to reduce the amount of gas, such as nitrous oxide, that is exhaled, wasted or vented into the atmosphere, and to reduce consumption of cylinder or pipeline gas.

### Background of the Invention

Medical gases are administered to patients in a number of different ways in different medical settings. For example, anaesthetic machines are typically sophisticated and expensive pieces of medical equipment, which are designed to administer a mixture of anaesthetic gases to the patient under the control of an anaesthetist. The patient is continuously monitored and the mixture of anaesthetic gases may be adjusted by the anaesthetist using the anaesthetic machine.

Ventilators are used to assist with a patient's breathing, typically in an intensive care unit under medical supervision. Ventilators usually deliver oxygen-enriched air under positive pressure into the patient's lungs for patients who are unable to breathe without assistance, though negative pressure ventilators may also be used.

Nitrous oxide is a widely used medical gas for use as a short-term rapid onset, rapid offset analgesic or sedative, as well as for inducing or co-inducing and maintaining anaesthesia during surgery. When used as an analgesic or sedative, nitrous oxide is typically administered as a mixture of 50% nitrous oxide and 50% oxygen (which is commercially available as Entonox^{®}, a registered trade mark of BOC Limited). A mixture of 50% nitrous oxide and 50% oxygen provides rapid onset of analgesia with minimal risk of inducing unconsciousness. Therefore, Entonox^{®} can be used safely in a variety of settings where short-term pain relief is required, and without close medical supervision. In some countries, nitrous oxide and oxygen may be administered via separate cylinders of pure gas - enabling bespoke, patient-specific gas ratios to be mixed prior to inhalation, although a fixed mixture of 50% nitrous oxide and 50% oxygen is typical in the art.

In some settings, Entonox^{®} may be self-administered by the patient in response to their pain level or in anticipation of a painful event. For example, in many countries, Entonox^{®} is used by women during labour to provide pain relief before and during a uterine contraction. Self-administration via a mask or a mouthpiece connected to a gas supply allows the woman to control her pain levels when she feels the onset of a contraction.

In order for Entonox^{®} to work rapidly and effectively, the patient must receive the gas mixture into their lungs, whereupon the nitrous oxide is rapidly absorbed into the bloodstream to provide pain relief. A mask used for administering Entonox^{®} may be equipped with an exhalation valve (such as the exhalation valve described in US 4,699,137) so that the patient can continuously breathe from an Entonox^{®} supply and exhale to atmosphere, whilst wearing the mask during the painful event.

Typically, a relatively high volume of nitrous oxide is consumed during labour. Since human metabolism of nitrous oxide is negligible, essentially all of this is subsequently released into the environment during exhalation or through wastage. Aside from the cost of consuming large volumes of nitrous oxide, release of nitrous oxide into the atmosphere is problematic for a number of reasons. Firstly, the concentration of nitrous oxide in a room in which the gas is being administered (e.g. delivery room) will increase over time unless nitrous oxide is ventilated, extracted or destroyed. Continuous exposure of staff to nitrous oxide, albeit at relatively low concentrations, is undesirable and possibly harmful over the long term. Secondly, nitrous oxide is a potent greenhouse gas, having approximately 300-fold more greenhouse potency than carbon dioxide, and there is increasing motivation to minimize release of nitrous oxide into the atmosphere via its use in medical settings.

One method for reducing nitrous oxide release is by means of a suitable gas destruction unit which may be connected to, for example, an exhalation valve of a mask used for administering Entonox^{®}. Current systems for destruction of nitrous oxide in medical settings typically employ catalytic splitters, which convert nitrous oxide into nitrogen and oxygen (see, for example, Ek, M., Tjus, K. Decreased emission of nitrous oxide from delivery wards-case study in Sweden. Mitig Adapt Strateg Glob Change 13, 809-818 (2008). https://doi.org/10.1007/s11027-008-9142-9). However, such systems are expensive and typically require exotic metal catalysts and/or the use of potentially dangerous elevated temperatures. Moreover, they are not well suited for handling the high gas flow rates associated with Entonox^{®} administration.

Anaesthetic machines use breathing circuits to administer nitrous oxide and other anaesthetic gases to patients. In a breathing circuit, the patient's exhaled breath may be purified using a carbon dioxide absorber (typically a soda lime cannister) and recycled back to the patient for inhalation. Anaesthetic breathing circuits are generally unsuitable for use outside an operating theatre or critical care environment, due to their necessary complexity, expense, and the skill level required to use them safely and effectively.

It would be desirable to provide a system for administering a mixture of nitrous oxide and oxygen to a patient, which provides sufficient concentration of the nitrous oxide to induce rapidly a desired effect, whilst minimizing release of the gas into the environment. It would be further desirable to minimize overall consumption of the gas, thereby reducing costs and minimizing transportation of cylinder gas both via a potentially polluting transport network, and within a medical facility.

WO 2017006186 A1 describes a system for delivering oxygen for breathing at a higher concentration than found in normal air, while reducing waste of oxygen gas.

WO 2016085807 A1 describes a closed-circuit breathing device that reduces loss of therapeutic gas to the atmosphere.

### Summary of the Invention

According to the present invention, there is provided an apparatus for administering gas to a patient, the apparatus comprising:
a respiratory part for delivering inhalation gas to the patient and receiving exhaled gas from the patient;
an inhalation line connected to the respiratory part, the inhalation line having an inhalation valve configured to be open during inhalation and closed during exhalation;
an exhalation line connected to the respiratory part, the exhalation line having an exhalation valve configured to be open during exhalation and closed during inhalation;
a gas supply line connected to the inhalation line, the gas supply line being connectable to a gas source;
a gas reservoir connected to the inhalation line;
a control valve having an inlet connected to the exhalation line and an outlet connected to the inhalation line, the control valve being configurable in a first position disconnecting the exhalation line and the inhalation line and a second position interconnecting the exhalation line and the inhalation line to form a breathing circuit;
a carbon dioxide absorber positioned in the breathing circuit; and
a control system configured for automatically switching the control valve from the first position to the second position,
wherein the control system comprises:
   an expandable chamber connected to the exhalation line for receiving exhaled gas, wherein expansion of the chamber causes the control valve to switch from the first position to the second position after a predetermined volume of exhaled gas has been received in the chamber.

For example, expansion of the chamber to a predetermined volume may be detected by a sensor (e.g. mechanical or electronic switch, optical sensor etc.), which, in turn, actuates the control valve to switch from the first position to the second position.

The apparatus described above advantageously enables delivery of gas (typically a mixture of oxygen and nitrous oxide) from the gas source to the patient's lungs and airway for an initial period when the control valve in its first position. During this initial period, the arrival of nitrous oxide into the lung establishes a concentration gradient which transfers the gas into arterial blood to provide rapid onset of a desired effect. Then, once a predetermined amount of exhaled gas has been exhaled via the exhalation line, the control valve switches to the second position allowing the patient to rebreathe exhaled gas via the breathing circuit comprising the interconnected exhalation and inhalation lines. Accordingly, the apparatus minimizes consumption of the gas from the gas source; minimizes an amount of exhaled gas released into the environment; provides rapid of onset of a desired pharmacological or physiological effect in the patient; and maintains the desired pharmacological or physiological effect throughout the patient's use of the apparatus. These and other advantages will be readily apparent to the person skilled in the art from the detailed description hereinbelow.

It will be further appreciated that the apparatus may be used for administering any type of gas (e.g. oxygen) to a patient, where a reduction in gas used may be beneficial, such as a pandemic response scenario where the supply of medical gases may come under pressure. Whilst useful for minimizing the use of medical gases, the apparatus is especially useful where rapid onset of a pharmacological or physiological effect is required. However, the apparatus is particularly suitable for administering an analgesic gas (e.g. a mixture of nitrous oxide and oxygen) to a patient, such as a woman during labour.

The control valve may be mechanically actuated and the control system further comprises a valve actuator operatively connected to the control valve. According to the invention, the chamber is configured to mechanically engage with the valve actuator after a predetermined volume of exhaled gas has been received in the chamber.

The apparatus according to the invention may advantageously obviate electronic systems, which require a power supply and typically add complexity and expense to the system. Furthermore, by avoiding the requirement for a power supply, the apparatus is highly portable and has no power supply cords attached or internal batteries requiring periodic replacement and/or recharging. Additionally, an apparatus lacking electronic systems may decrease the risk of fire and or explosion when using a flammable or oxidizing gas.

The valve actuator and/or the chamber may be adjustable for adjusting the volume of exhaled gas required for switching the control valve from the first position to the second position. For example, patients with a relatively smaller lung capacity (e.g. children, elderly patients, patients with impaired lung function etc) may benefit from adjustment of the valve actuator such that a relatively smaller volume of exhaled gas is required to switch the control valve from the first position to the second position, as compared to patients with a relatively larger lung capacity.

Typically, the expandable chamber is a bellows chamber. Alternatively, the expandable chamber may be another type of flexible chamber, bottle, bag or pillow, which may be formed of a polymer material or other suitable material known to persons skilled in the art.

The gas reservoir may be filled with purified exhaled gas when the control valve is in the second position, and the patient rebreathes the purified exhaled gas from the gas reservoir. The gas reservoir is typically an expandable bag, such as a balloon. Typically, the carbon dioxide absorber comprises a soda lime or other means of absorbing/removing carbon dioxide so as to purify exhaled gas. For example, the carbon dioxide absorber may comprise lithium hydroxide or photosynthesizing biological materials, or may be in the form of another suitable container or reactor positioned in the inhalation line upstream of the gas reservoir.

The gas supply line may comprise a demand valve for regulating gas flow into the inhalation line during inhalation, the demand valve preferentially allowing rebreathing of the purified exhaled gas from the gas reservoir when the gas reservoir contains the purified exhaled gas. Accordingly, the patient rebreathes at least some exhaled gas whenever the gas reservoir contains purified exhaled gas suitable for rebreathing.

The gas source may be, for example, a replaceable gas cylinder detachably connected to the gas supply line, or a piped gas supply detachably connected to the gas supply line. The demand valve may be integrated into the gas supply line or, alternatively, may be part of a gas cylinder assembly specifically adapted for delivering medical gas on demand. These and other arrangements for regulating a supply of gas from the gas source will be readily apparent to the person skilled in the art.

The exhalation line may have a vent for venting exhaled gas. The vent may be connected to a suitable gas scrubber or scavenging system to minimize any exhaled gas vented from the apparatus being released into the atmosphere - or otherwise vent gas to the atmosphere without removal or treatment of gas to minimize exposure of healthcare staff to potentially deleterious compounds. In some examples, the vent comprises an adjustable pressure-limiting (APL) valve, such as those used in anaesthetic machines, optionally in combination with a fixed or adjustable orifice. An APL valve advantageously controls an amount of gas released from the system without ingress of ambient air. Typically, the vent is configured to vent from 5 to 20% of a volume of exhaled gas for each breath by the patient, such that, during idle periods, the apparatus passively resets by venting exhaled gas from the expandable chamber. A period of time taken for the apparatus to reset may be controlled using the APL valve, depending, for example, on the frequency of a painful event (e.g. contraction) experienced by the patient.

The respiratory part may comprise at least one of:
a patient gas line commonly connected at one end to the inhalation line and the exhalation line; and
a respiratory interface selected from the group consisting of: a mouthpiece, a mask and a nosepiece.

The patient gas line and/or the respiratory interface may include a bacterial/viral filter. For example, the bacterial/viral filter may be incorporated into a single-use, replaceable respiratory interface detachably connected to the patient gas line, while the patient gas line is typically a multi-patient-use part of the apparatus.

Alternatively, the respiratory interface may be connected directly to both the inhalation line and the exhalation line, although this arrangement requires two connections to the respiratory interface engaged with the patient.

As used herein, the term "gas" refers to any gas or mixtures of gases. For example, the gas supplied by the gas source may be a mixture of nitrous oxide and oxygen (e.g. 50/50 mixture), pure oxygen, oxygen-enriched air etc. As indicated by the context, and unless specified otherwise, the term "gas" may refer to any gas or mixture of gases contained in the apparatus at any given time, such as gas supplied from a dedicated gas source, gas inhaled by a patient, gas exhaled by a patient, gas purified in the breathing circuit, gas vented from the apparatus etc.

As used herein, the term "patient" refers to any subject receiving gas, whether or not in a hospital setting. Accordingly, a "patient" may refer to a hospital patient receiving a medical treatment, a woman in labour, a person receiving dental treatment, and/or any other person requiring gas administration to provide, for example, pain relief, sedation, breathing assistance etc.

For the avoidance of doubt, the term "comprising", or variations such as "comprise" or "comprises", should be construed as including a stated element, integer or step, but not excluding any other element, integer or step.

Likewise, for the avoidance of doubt, the term "a" (or "an"), in phrases such as "comprising a", should be taken to mean "at least one" and not "one and only one". Where the term "at least one" is specifically used, this should not be construed as having any limitation on the definition of "a".

### Brief Description of the Drawings

Embodiments of the present disclosure will now be described by way of example only with reference to the accompanying drawings, in which:
Figure 1A shows schematically an apparatus according to the present invention in a quiescent state;
Figure 1B shows schematically the apparatus according to the present invention after an initial patient exhalation;
Figure 1C shows schematically the apparatus according to the present invention after a predetermined amount of exhaled gas has been exhaled via the exhalation line;
Figure 2A shows schematically an apparatus according to an example during an initial patient exhalation; and
Figure 2B shows schematically the apparatus according to an example after a predetermined amount of exhaled gas has been exhaled via the exhalation line.

### Detailed Description of the Invention

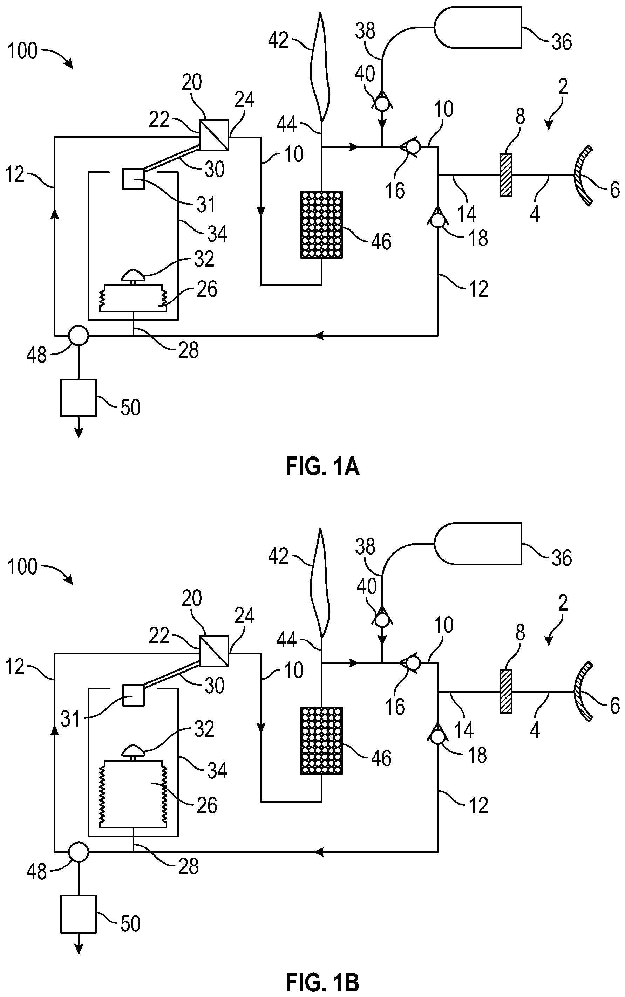

Referring to Figure 1A, there is shown schematically an apparatus 100 according to the present invention for administering gas to a patient. The apparatus 100 comprises a respiratory part 2, which includes a patient gas line 4 connected at one end to a respiratory interface 6 for engagement with the patient. The patient gas line 4 typically takes the form of flexible polymer tubing having an internal diameter sufficient for handling gas flow rates associated with a particular use case. For example, the patient gas line 4 may be corrugated tubing having an internal diameter of 10 to 30 mm for handling relatively high gas flow rates used in administration of Entonox^{®} to women during labour.

The respiratory interface 6 typically comprises a conventional mask, a mouthpiece or a nosepiece, which is detachably connected to the patient gas line 4. Usually, the respiratory interface 6 is disposable and replaced for each patient after use. For example, the respiratory interface 6 may be comprised of biodegradable material. The patient gas line 4 and/or the respiratory interface 6 includes an inline filter 8 for filtering viral and/or bacterial particles, thereby minimizing cross-contamination between patients.

The patient gas line 4 is commonly connected to an inhalation line 10 and an exhalation line 12 at an opposite end from the respiratory interface 6. Accordingly, the inhalation line 10, the exhalation line 12 and the patient gas line 4 are all in fluid communication and meet together at a T-junction 14. The patient gas line 4 allows two-way gas flow to and from the patient - inhalation gas is received at the T-junction 14 from the inhalation line 10 and flows through the patient gas line 4 towards the respiratory interface 6 for inspiration by the patient; and gas exhaled by the patient is received from the respiratory interface and flows through the patient gas line towards the T-junction and into the exhalation line 12.

The directionality of gas flow through the inhalation and exhalation lines 10 and 12 is controlled by one-way (non-return) valves. A one-way inhalation valve 16 is positioned in the inhalation line 10 upstream of the T-junction 14 and is configured to be open during inhalation and closed during exhalation. Similarly, a one-way exhalation valve 18 is positioned in the exhalation line 12 downstream of the T-junction 14 and is configured to be open during exhalation and closed during inhalation. The direction of gas flow is indicated by arrows in the inhalation and exhalation lines 10 and 12 in Figure 1A.

The inhalation line 10 and the exhalation line 12 extend from the T-junction 14 and meet at a mechanically-actuated first control valve 20. The inhalation and exhalation lines 10 and 12 may be comprised of or internally coated with copper to provide self-sterilization.

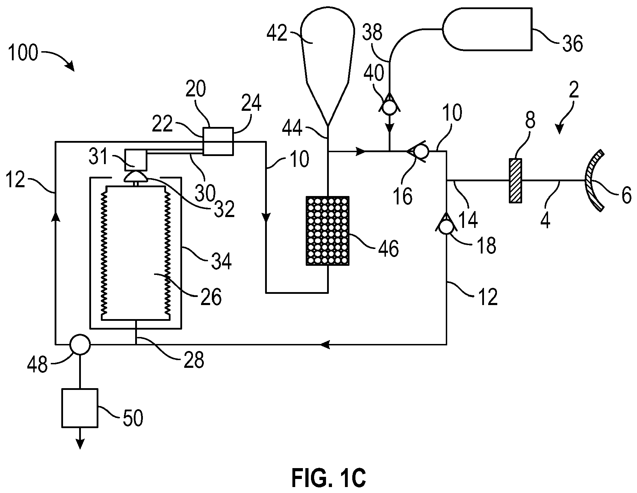

The first control valve 20 has an inlet 22 connected to the exhalation line 12 and an outlet 24 connected to the inhalation line 10, and is configurable in first and second positions. In the first position shown in Figure 1A, the first control valve 20 is closed with the exhalation line 12 disconnected from the inhalation line 10. In this first position, gas exhaled from the patient flows from the exhalation line 12 into an expandable bellows chamber 26, which is connected to the exhalation line via a control line 28. In the second position shown in Figure 1C, the first control valve 20 is open such that the exhalation line 12 and the inhalation line 10 are interconnected to form a breathing circuit.

Suitable mechanically-actuated control valves will be well known to the person skilled in the art. For example, a simple fill valve or variant thereof, may be suitable for use in the apparatus 100 according to the present invention. It will be further appreciated that perfect gas sealing of the first control valve 20 is not necessary for the apparatus 100 to function satisfactorily.

Switching of the first control valve 20 from the first position to the second position is by means of a valve actuator 30 in the form of a swing arm operatively connected to the first control valve. The valve actuator 30 has an end portion 31 positioned for mechanical engagement with an engagement surface 32 of the bellows chamber 26. With the bellows chamber 26 empty, the first control valve 20 is configured in its first position, which allows the bellows chamber to fill with exhaled gas from the exhalation line 12. Once the bellows chamber 26 has been filled with the predetermined volume of exhaled gas (Figure 1C), the engagement surface 32 of the bellows chamber engages with the end portion 31 of the valve actuator 30 so that the valve actuator swings in the direction of the expanding bellows chamber, causing the first control valve 20 to switch from the first position to the second position. With contraction of bellows chamber 26, the valve actuator 30 swings back in the direction of the contracting bellows chamber, causing the first control valve 20 to switch back to its first position. Typically, the bellows chamber 26 and at least part of the valve actuator 30 are contained in a suitable housing 34.

The inhalation line 10, extending from the outlet 24 of the first control valve 20 to the T-junction 14, enables rebreathing of exhaled gas and/or inspiration of gas from a dedicated gas source 36. A gas supply line 38 is connected at one end to the inhalation line 10 upstream of the T-junction 14. An opposite end of the gas supply line 38 is connected to the gas source 36, which may be a gas cylinder (e.g. Entonox^{®} cylinder) as shown in Figures 1A-C or a piped gas supply. As is known in the art, a demand valve 40 in the gas supply line 38 regulates a supply of gas from the gas source 36 to the patient.

A gas reservoir in the form of an expandable reservoir bag 42 (e.g. balloon or bellows) is connected to the inhalation line 10 via a reservoir line 44 upstream of the gas supply line 38. An inline carbon dioxide absorber, such as a soda lime cannister 46, is positioned in the inhalation line 10 upstream of the reservoir bag 42 between the first control valve 20 and the reservoir line 44.

With the first control valve 20 in its first position and the reservoir bag 42 empty, as shown in Figure 1A, a patient's first inhalation is from the gas source 36, which by way of example, contains a 50/50 mixture of nitrous oxide and oxygen (Entonox^{®}). Thus, the patient is ensured a high dosage of inspired Entonox^{®} from the gas supply line 38 with the first inhalation. Most of the nitrous oxide is absorbed into the patient's bloodstream from the first inhalation meaning that the patient's subsequent first exhalation contains very little nitrous oxide together with nitrogen (contained in the patient's lungs before the first inhalation), carbon dioxide and some unmetabolized oxygen. Since the first control valve 20 is in its first position, the exhaled gas partially fills the bellow chamber 28 via the exhalation line 12, as shown in Figure 1B.

With the patient's next inhalation, the first control valve 20 remains in the first position shown in Figure 1B and the inhaled gas once again is delivered entirely from the gas source 36 containing Entonox^{®}. The patient once again receives a high dosage of inspired Entonox^{®} and will begin to experience rapid onset of its analgesic and sedative effects. The patient's bloodstream, having already absorbed a relatively high dosage of nitrous oxide from the first inhalation, will absorb a somewhat smaller amount of nitrous oxide from the second inhalation. Therefore, exhaled gas from the patient's second exhalation will contain a higher concentration of nitrous oxide than the first exhalation, as well as carbon dioxide and unmetabolized oxygen which continues to fill the bellows chamber 26.

With each inhalation and exhalation cycle, the bellows chamber 26 will continue to fill with exhaled gas, and the exhaled gas will contain increasingly higher concentrations of nitrous oxide since the patient's bloodstream will typically approach an equilibrium concentration of nitrous oxide after about three inspirations of pure Entonox^{®}.

As foreshadowed above, once the bellows chamber 26 has filled with a predetermined volume of exhaled gas, the engagement surface 32 of the bellows chamber butts against part of the valve actuator 30 to switch the first control valve 20 from the first position (Figure 1B) to the second position (Figure 1C). The predetermined volume of exhaled gas required for actuation of the first control valve 20 may be in the range of 3 to 20 litres or 5 to 15 litres.

It will be appreciated that the predetermined volume of exhaled gas required to fill the bellows chamber sufficiently for actuating the first control valve 20 may be varied by adjusting a configuration or position of the valve actuator 30 and/or the engagement surface 23. For example, some patients may exhale about 3 litres of gas with each breath whilst experiencing a painful episode. Therefore, the bellows chamber 26 may be configured to have a capacity of about 9 litres such that the first control valve 20 is actuated to switch to its second position after three exhalations. For other patients, the valve actuator 30 and/or engagement surface 32 may be adjusted such that a relatively smaller volume of gas is required to actuate the first control valve 20. For example, the engagement surface 32 may mounted on an extendible stem to adjust the volume of exhaled gas required to effect actuation of the first control valve 20. Other means for adjusting this required volume will be readily apparent to the person skilled in the art.

With the first control valve 20 in its second position, as shown in Figure 1C, exhaled gas now enters the inhalation line 10 and is first purified by passing through the soda lime cannister 46 to remove carbon dioxide. This purified exhaled gas then fills the reservoir bag 42 and is held in the reservoir bag by virtue of the one-way inhalation and exhalation valves 16 and 18. The patient's next inhalation via the patient gas line 4 will then preferentially consume the purified exhaled gas held in the reservoir bag 42 by virtue of a lower cracking pressure of the inhalation line valve 16 compared to the demand valve 40 in the gas supply line 38. The reservoir bag 42 deflates until the next exhalation whereupon the reservoir bag will reinflate with purified exhaled gas. The patient is now rebreathing mostly exhaled gas via the breathing circuit and consuming only a small amount of gas from the gas source 36. Since the patient's blood is approaching an equilibrium concentration of nitrous oxide after about three breaths, the exhaled gas entering the breathing circuit contains a relatively high concentration of nitrous oxide, which is sufficient to maintain the required level of analgesia. On the other hand, if the patient were to rebreathe exhaled gas immediately after the first inspiration of Entonox^{®}, then the second and third breaths would contain insufficient concentrations of nitrous oxide to induce analgesia.

Still referring to Figures 1A-C, the exhalation line 12 has a vent comprising an adjustable pressure-limiting (APL) valve 48, which provide a gas path from the bellows 26 to a region external to the system and is used for resetting the apparatus 100, as well as allowing a quantity of fresh gas from the gas source 36 to be introduced into the breathing circuit. The APL valve 48 allows exhaled gas contained in the bellows chamber 26 and/or the exhalation line 12 to be vented from the apparatus 100 in a controllable manner. Typically, the APL valve 48 is set to release about 5 to 20% (e.g. about 10%) of a volume of each exhaled breath from the exhalation line 12 over a period of one breathing cycle. Therefore, even when the patient is rebreathing exhaled gas from the reservoir bag 42, the patient will still receive at least some quantity (e.g. 5 to 20%) of fresh gas from the gas source 36 during each inhalation. In practice, the bellows chamber 26 contracts somewhat after each exhalation via venting of some exhaled gas contained therein through the APL valve 48. Contraction of the bellows chambers 26 will cause the first control valve 20 to switch back to the first position before or during the next inhalation. Regardless of the position of the first control valve 20 during inhalation, the patient's inhalation gas will be delivered mostly from the reservoir bag 42 and partially from the gas source 36 once the apparatus is primed for rebreathing.

The APL valve 48 also serves the important function of passively resetting the apparatus 100 during idle periods when the patient is not breathing via the respiratory interface 6. Typically, the APL valve 48 is adjusted to allow all gas contained in the bellows chamber 26 to be vented from the apparatus over a time period of about 1 to 5 minutes. However, this time period may be varied, as required, by adjusting the APL valve 48 or by causing the gas to pass through a fixed or adjustable orifice. For example, when a patient experiences more frequent painful episodes (e.g. towards the end of labour), then the APL valve 48 may be opened further to shorten the time period required for emptying the bellows chamber 26 - or a restricting orifice in the gas path may be enlarged.

As shown in Figures 1A-C, the APL valve 48 may be vented through a gas scrubber 50, such as a nitrous oxide destruction unit, in order to minimize an amount of nitrous oxide released to atmosphere. Suitable gas scrubbers will be well known to the person skilled in the art. Advantageously, the gas scrubber 50 is not required to handle very high gas flow rates in contrast with systems that connect gas scrubbers directly to an exhalation valve of a mask. By way of example, the gas scrubber 50 may only be required to split 1 to 2 litres of nitrous oxide per minute instead of 1 to 2 litres of nitrous oxide every 10 seconds in the case of a conventional Entonox^{®} delivery system. A 5-fold or greater reduction in the flow rate of nitrous oxide through the gas scrubber has significant advantages for the design and operation of a catalytic destruction unit, typically used as the nitrous oxide gas scrubber.

From the foregoing, it will be appreciated that the apparatus 100 according to the present invention advantageously achieves rapid onset of analgesia, minimizes an amount of nitrous oxide consumed by the patient and, consequently, minimizes an amount of nitrous oxide released to atmosphere. Compared to conventional gas administration, the apparatus according to the present invention may save up to 50%, up to 75% or up to 90% of gas administered via conventional masks, whilst providing comparable physiological or pharmacological effects.

It is a further advantage that the apparatus according to the present invention is inexpensive and contains no electronic components meaning that it is highly portable and can be used in virtually any setting. It is a further advantage that, from the patient's perspective, rebreathing exhaled gas via the breathing circuit is a more comfortable experience than breathing pure gas directly from the gas source 36, because the rebreathed gas is relatively warm and humid.

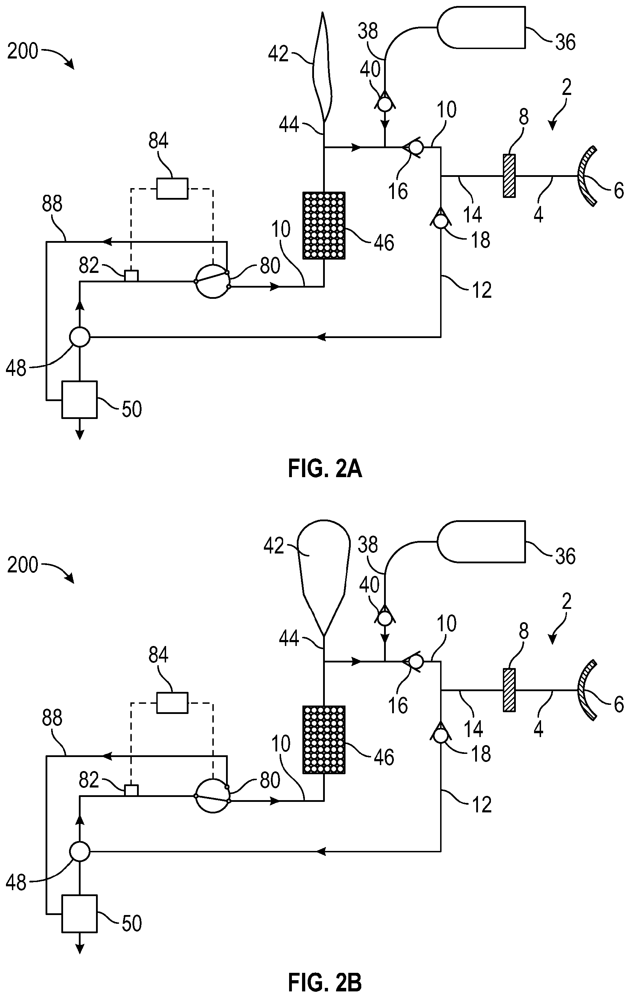

Referring to Figures 2A and 2B, there is shown schematically an apparatus 200 for administering gas to a patient according to an example. In the interests of clarity, like reference numerals are used to indicated like features, where relevant, in the embodiment of the invention and the example

The apparatus 200 according to the example functions in a similar manner to the apparatus 100 according to the invention described above in connection with Figures 1A-C. The patient initially receives inhalation gas from a dedicated gas source 36 and then the apparatus 200 switches to the breathing circuit after a predetermined amount of gas has been exhaled via the exhalation line 12. However, instead of using the mechanically-actuated first control valve 20 in combination with the expandable bellows chamber 26 engaged with the valve actuator 30, the apparatus 200 according to the example employs an electronically-actuated second control valve 80, such as a three-way solenoid valve, in combination with a sensor 82 and a controller 84.

As shown in Figures 2A and 2B, the sensor 82 is positioned for sensing exhaled gas in the exhalation line 12, although it will be appreciated the sensor 82 may be positioned at any part of the circuit for sensing exhaled gas. For example, the sensor 82 may be a flow meter or a manometer for sensing a gas flow rate or a pressure of exhaled gas in the exhalation line 12 or a sensor configured for measuring a concentration of a specific gas, for example, an end tidal nitrous oxide measurement. The sensor 82 communicates a control signal back to the controller 84, the control signal being indicative of a number of exhalations by the patient. For example, a rapid rise and fall in a gas flow rate or gas pressure in the exhalation line 12 will be indicative one exhalation. Once the controller 84 has determined, using the control signal, that the patient has exhaled a predetermined number of times, or has reached a particular end tidal nitrous oxide concentration, then the controller signals to the second control valve 80 to switch from the first position (Figure 2A) to the second position (Figure 2B).

In the first position shown in Figure 2A, the second control valve 80 is configured to vent exhaled gas received in the exhalation line 12 into the gas scrubber 50 via a vent line 88, and the patient inhales gas directly from the gas source 36 via the inhalation line 10. In the second position shown in Figure 2B, the second control valve 80 is configured to form the breathing circuit by interconnecting the exhalation line 12 and the inhalation line 10. The controller 84 maintains the second control valve 80 in the second position until such time that a control signal from the sensor 82 stops indicating that the patient is exhaling via the exhalation line 12. Once the patient has ceased breathing through the patient interface 6 for a period of time (e.g. 10 to 30 seconds), then the controller 84 will automatically switch the second control valve 80 from the second position to the first position, thereby resetting the apparatus 200 for its next use.

All other functions of the apparatus 200 according to the example are essentially the same as those described above in connection with Figures 1A-C. The APL valve 48 is connected to the exhalation line 12 to allow an amount of fresh gas from the gas source 36 to be introduced into the breathing circuit when the patient is rebreathing exhaled gas from the gas reservoir 42, although the same affect may be achieved by switching position of the solenoid valve 80 after a particular volume of gas has been measured - for example between 70 and 95% of the inhaled or exhaled volume. However, the APL valve 48 is not required to reset the apparatus 200, because the bellows chamber 26 is redundant in this example.

The apparatus 200 according to the example advantageously enables more accurate control of switching of the second control valve 80 in response to a patient's exhalations, as well as automatic resetting of the apparatus between uses. However, the apparatus 200 according to the example has the disadvantage of requiring a power supply, microelectronic circuitry in the controller 84 and a solenoid valve.

The skilled person will appreciate that other variants of the apparatus described above are within the ambit of the present disclosure, based on the principle of a control system which is configured to switch a control valve from the first position to the second position after a predetermined amount of exhaled gas has been exhaled via the exhalation line 12, or a specific end tidal nitrous oxide concentration has been reached. For example, a hybrid system may employ the bellows chamber 26 with mechanical actuation of an electronic switch controlling the solenoid control valve 80 of the example. Alternatively, or in addition, gas supply line 38 can be connected at one end to the inhalation line 10 upstream, and optionally immediately upstream, of the inline carbon dioxide absorber (i.e., soda lime cannister 46), ensuring that the carbon dioxide absorber is primed with fresh Entonox^{®}.

It will further be appreciated that, while the apparatus described herein is especially useful for administering an analgesic gas to a patient, it may be used for administration of any gas. For example, the apparatus may be used as a low-cost alternative for administering oxygen to patients, especially in scenarios where oxygen is in short supply.

The present invention is defined by the accompanying claims.

## Claims

1. An apparatus (100) for administering gas to a patient, the apparatus (100) comprising:
a respiratory part (2) for delivering inhalation gas to the patient and receiving exhaled gas from the patient;
an inhalation line (10) connected to the respiratory part (2), the inhalation line (10) having an inhalation valve (16) configured to be open during inhalation and closed during exhalation;
an exhalation line (12) connected to the respiratory part (2), the exhalation line (12) having an exhalation valve (18) configured to be open during exhalation and closed during inhalation;
a gas supply line (38) connected to the inhalation line (10), the gas supply line (38) being connectable to a gas source (36);
a gas reservoir (42) connected to the inhalation line (10);
a control valve (20) having an inlet connected to the exhalation line (12) and an outlet connected to the inhalation line (10), the control valve (20) being configurable in:
a first position disconnecting the exhalation line (12) and the inhalation line (10), and
a second position interconnecting the exhalation line (12) and the inhalation line (10) to form a breathing circuit; and
a carbon dioxide absorber (46) positioned in the breathing circuit;
the apparatus (100) comprising a control system configured for automatically switching the control valve (20) from the first position to the second position,
**characterized in that**
the control system comprises an expandable chamber (26) connected to the exhalation line (12) for receiving exhaled gas, wherein expansion of the chamber (26) causes the control valve (20) to switch from the first position to the second position after a predetermined volume of exhaled gas has been received in the chamber (26).

2. The apparatus (100) of claim 1, wherein the control system further comprises a valve actuator (30) operatively connected to the control valve (20), wherein the chamber (26) is configured to engage with the valve actuator (30) after a predetermined volume of exhaled gas has been received in the chamber (26).

3. The apparatus (100) of claim 2, wherein the control valve (20) is mechanically actuated by the valve actuator (30).

4. The apparatus (100) of any preceding claim, wherein the chamber (26) is a bellows chamber.

5. The apparatus (100) of any one of the preceding claims, wherein the gas reservoir (42) is filled with purified exhaled gas when the control valve (20) is in the second position, and the patient rebreathes the purified exhaled gas from the gas reservoir (42).

6. The apparatus (100) of claim 5, wherein the gas supply line (38) comprises a demand valve (40) for regulating gas flow into the inhalation line (10) during inhalation, the demand valve (40) preferentially allowing rebreathing of the purified exhaled gas from the gas reservoir (42) when the gas reservoir (42) contains the purified exhaled gas.

7. The apparatus (100) of any one of the preceding claims, wherein the exhalation line (12) has a vent (48) for venting exhaled gas.

8. The apparatus (100) of claim 7, wherein the vent (48) comprises an adjustable pressure-limiting valve.

9. The apparatus (100) of claim 7 or 8, wherein the vent (48) is configured to vent (48) from 5 to 20% of a volume of exhaled gas for each breath by the patient.

10. The apparatus (100) of claims 7 to 9, wherein, during idle periods, the apparatus (100) passively resets by venting exhaled gas.

11. The apparatus (100) of any one of the preceding claims, wherein the respiratory part (2) comprises at least one of:
a patient gas line (4) commonly connected at one end to the inhalation line (10) and the exhalation line (12); and
a respiratory interface (6) for engagement with the patient, the respiratory interface (6) being selected from the group consisting of: a mouthpiece, a mask and a nosepiece.

12. A system for administering gas to a patient, the system comprising the apparatus (100) according to any one of the preceding claims and one or more of:
a gas source (36) connected to the gas supply line (38);
a respiratory interface (6) for engagement with the patient, the respiratory interface (6) being selected from the group consisting of: a mouthpiece, a mask and a nosepiece; and
a gas scrubber (50) connected to the vent (48).

## Patentansprüche

1. Eine Vorrichtung (100) zum Verabreichen von Gas an einen Patienten, wobei die Vorrichtung (100) Folgendes beinhaltet:
einen respiratorischen Teil (2) zum Zuführen von Inhalationsgas zu dem Patienten und zum Aufnehmen von ausgeatmetem Gas von dem Patienten;
eine Inhalationsleitung (10), die mit dem respiratorischen Teil (2) verbunden ist, wobei die Inhalationsleitung (10) ein Inhalationsventil (16) aufweist, das dazu ausgelegt ist, während der Inhalation offen zu sein und während der Exhalation geschlossen zu sein;
eine Exhalationsleitung (12), die mit dem respiratorischen Teil (2) verbunden ist, wobei die Exhalationsleitung (12) ein Exhalationsventil (18) aufweist, das dazu ausgelegt ist, während der Exhalation offen zu sein und während der Inhalation geschlossen zu sein;
eine Gaszuleitung (38), die mit der Inhalationsleitung (10) verbunden ist, wobei die Gaszuleitung (38) mit einer Gasquelle (36) verbunden werden kann;
ein Gasreservoir (42), das mit der Inhalationsleitung (10) verbunden ist;
ein Steuerventil (20), das einen mit der Exhalationsleitung (12) verbundenen Einlass und einen mit der Inhalationsleitung (10) verbundenen Auslass aufweist, wobei das Steuerventil (20) dazu ausgelegt sein kann, in:
einer ersten Position zu sein, wodurch die Verbindung von Exhalationsleitung (12) und Inhalationsleitung (10) getrennt wird, und
einer zweiten Position zu sein, wodurch die Exhalationsleitung (12) und die Inhalationsleitung (10) miteinander verbunden werden, um einen Atmungskreislauf zu bilden; und
einen Kohlendioxidabsorber (46), der in dem Atmungskreislauf positioniert ist;
wobei die Vorrichtung (100) ein Steuersystem beinhaltet, das dazu ausgelegt ist, das Steuerventil (20) automatisch von der ersten Position zu der zweiten Position umzuschalten, **dadurch gekennzeichnet, dass** das Steuersystem eine expandierbare Kammer (26) beinhaltet, die mit der Exhalationsleitung (12) verbunden ist, um ausgeatmetes Gas aufzunehmen, wobei die Expansion der Kammer (26) das Steuerventil (20) veranlasst von der ersten Position zu der zweiten Position umzuschalten, nachdem ein zuvor bestimmtes Volumen von ausgeatmetem Gas in der Kammer aufgenommen worden ist (26).

2. Vorrichtung (100) gemäß Anspruch 1, wobei das Steuersystem ferner ein Ventilbetätigungsglied (30) beinhaltet, das betriebsfähig mit dem Steuerventil (20) verbunden ist, wobei die Kammer (26) dazu ausgelegt ist, in Eingriff mit dem Ventilbetätigungsglied (30) zu kommen, nachdem ein zuvor bestimmtes Volumen von ausgeatmetem Gas in der Kammer (26) aufgenommen worden ist.

3. Vorrichtung (100) gemäß Anspruch 2, wobei das Steuerventil (20) durch das Ventilbetätigungsglied (30) mechanisch betätigt wird.

4. Vorrichtung (100) gemäß einem der vorhergehenden Ansprüche, wobei die Kammer (26) eine Balgkammer ist.

5. Vorrichtung (100) gemäß einem der vorhergehenden Ansprüche, wobei das Gasreservoir (42) mit gereinigtem ausgeatmetem Gas gefüllt ist, wenn das Steuerventil (20) sich in der zweiten Position befindet, und der Patient das gereinigte ausgeatmete Gas aus dem Gasreservoir (42) erneut einatmet.

6. Vorrichtung (100) gemäß Anspruch 5, wobei die Gaszuleitung (38) ein Bedarfsventil (40) zum Regulieren des Gasflusses in die Inhalationsleitung (10) während der Inhalation beinhaltet, wobei das Bedarfsventil (40) bevorzugt das erneute Einatmen des gereinigten ausgeatmeten Gases aus dem Gasreservoir (42) erlaubt, wenn das Gasreservoir (42) das gereinigte ausgeatmete Gas enthält.

7. Vorrichtung (100) gemäß einem der vorhergehenden Ansprüche, wobei die Exhalationsleitung (12) eine Entlüftung (48) zum Entlüften von ausgeatmetem Gas aufweist.

8. Vorrichtung (100) gemäß Anspruch 7, wobei die Entlüftung (48) ein einstellbares Druckminderventil beinhaltet.

9. Vorrichtung (100) gemäß Anspruch 7 oder 8, wobei die Entlüftung (48) zum Entlüften (48) von 5 bis 20% eines Volumens von ausgeatmetem Gas für jeden Atemzug durch den Patienten ausgelegt ist.

10. Vorrichtung (100) gemäß Ansprüchen 7 bis 9, wobei sich die Vorrichtung (100) während der Ruhezeiten durch das Entlüften von ausgeatmetem Gas passiv zurückstellt.

11. Vorrichtung (100) gemäß einem der vorhergehenden Ansprüche, wobei der respiratorische Teil (2) mindestens eines der Folgenden beinhaltet:
eine Patientengasleitung (4), die an einem Ende gemeinsam mit der Inhalationsleitung (10) und der Exhalationsleitung (12) verbunden ist; und
eine respiratorische Grenzfläche (6) zum In-Eingriff-Kommen mit dem Patienten, wobei die respiratorische Grenzfläche (6) aus der Gruppe ausgewählt ist, die aus Folgenden besteht: einem Mundstück, einer Maske und einem Nasenstück.

12. Ein System zum Verabreichen von Gas an einen Patienten, wobei das System die Vorrichtung (100) nach einem der vorhergehenden Ansprüche und eines oder mehrere der Folgenden beinhaltet:
eine Gasquelle (36), die mit der Gaszuleitung (38) verbunden ist;
eine respiratorische Grenzfläche (6) zum In-Eingriff-Kommen mit dem Patienten, wobei die respiratorische Grenzfläche (6) aus der Gruppe ausgewählt ist, die aus Folgenden besteht: einem Mundstück, einer Maske und einem Nasenstück; und
einen Gaswäscher (50), der mit der Entlüftung (48) verbunden ist.

## Revendications

1. Appareil (100) pour administrer du gaz à un patient, l'appareil (100) comprenant :
une partie respiratoire (2) pour délivrer du gaz d'inspiration au patient et recevoir du gaz expiré du patient ;
une conduite d'inspiration (10) connectée à la partie respiratoire (2), la conduite d'inspiration (10) ayant une soupape d'inspiration (16) configurée pour être ouverte pendant l'inspiration et fermée pendant l'expiration ;
une conduite d'expiration (12) connectée à la partie respiratoire (2), la conduite d'expiration (12) ayant une soupape d'expiration (18) configurée pour être ouverte pendant l'expiration et fermée pendant l'inspiration ;
une conduite d'alimentation en gaz (38) connectée à la conduite d'inspiration (10), la conduite d'alimentation en gaz (38) pouvant être connectée à une source de gaz (36) ;
un réservoir de gaz (42) connecté à la conduite d'inspiration (10) ;
une soupape de commande (20) ayant une entrée connectée à la conduite d'expiration (12) et une sortie connectée à la conduite d'inspiration (10), la soupape de commande (20) pouvant être configurée en :
une première position déconnectant la conduite d'expiration (12) et la conduite d'inspiration (10), et
une deuxième position interconnectant la conduite d'expiration (12) et la conduite d'inspiration (10) pour former un circuit de respiration ; et
un absorbeur de dioxyde de carbone (46) positionné dans le circuit de respiration ;
l'appareil (100) comprenant un système de commande configuré pour faire passer automatiquement la soupape de commande (20) de la première position à la deuxième position, **caractérisé en ce que** le système de commande comprend une chambre expansible (26) connectée à la conduite d'expiration (12) pour recevoir du gaz expiré, où l'expansion de la chambre (26) amène la soupape de commande (20) à passer de la première position à la deuxième position après qu'un volume prédéterminé de gaz expiré a été reçu dans la chambre (26).

2. Appareil (100) selon la revendication 1, où le système de commande comprend en outre un actionneur de soupape (30) connecté de manière opérationnelle à la soupape de commande (20), où la chambre (26) est configurée pour s'engager avec l'actionneur de soupape (30) après qu'un volume prédéterminé de gaz expiré a été reçu dans la chambre (26).

3. Appareil (100) selon la revendication 2, où la soupape de commande (20) est actionnée mécaniquement par l'actionneur de soupape (30).

4. Appareil (100) selon l'une quelconque des revendications précédentes, où la chambre (26) est une chambre à soufflet.

5. Appareil (100) selon l'une quelconque des revendications précédentes, où le réservoir de gaz (42) est rempli de gaz expiré purifié lorsque la soupape de commande (20) est dans la deuxième position, et le patient réinhale le gaz expiré purifié à partir du réservoir de gaz (42).

6. Appareil (100) selon la revendication 5, où la conduite d'alimentation en gaz (38) comprend une soupape de demande (40) pour réguler le débit de gaz dans la conduite d'inspiration (10) pendant l'inspiration, la soupape de demande (40) permettant de préférence la réinhalation du gaz expiré purifié à partir du réservoir de gaz (42) lorsque le réservoir de gaz (42) contient le gaz expiré purifié.

7. Appareil (100) selon l'une quelconque des revendications précédentes, où la conduite d'expiration (12) comporte un évent (48) pour l'évacuation de gaz expiré.

8. Appareil (100) selon la revendication 7, où l'évent (48) comprend une soupape de limitation de pression réglable.

9. Appareil (100) selon la revendication 7 ou 8, où l'évent (48) est configuré pour évacuer (48) de 5 à 20 % d'un volume de gaz expiré pour chaque respiration du patient.

10. Appareil (100) selon les revendications 7 à 9, où, pendant les périodes d'inactivité, l'appareil (100) se réinitialise passivement en évacuant du gaz expiré.

11. Appareil (100) selon l'une quelconque des revendications précédentes, où la partie respiratoire (2) comprend au moins un parmi :
une conduite de gaz patient (4) communément connectée à une extrémité à la conduite d'inspiration (10) et à la conduite d'expiration (12) ; et
une interface respiratoire (6) pour l'engagement avec le patient, l'interface respiratoire (6) étant choisie dans le groupe consistant en : un embout buccal, un masque et un embout nasal.

12. Système pour administrer du gaz à un patient, le système comprenant l'appareil (100) selon l'une quelconque des revendications précédentes et un ou plusieurs parmi :
une source de gaz (36) connectée à la conduite d'alimentation en gaz (38) ;
une interface respiratoire (6) pour l'engagement avec le patient, l'interface respiratoire (6) étant choisie dans le groupe consistant en : un embout buccal, un masque et un embout nasal ; et
un épurateur de gaz (50) connecté à l'évent (48).
